# EUROPÄISCHE PATENTSCHRIFT

(11) **EP 2 135 546 B1**
(45) Veröffentlichungstag und Bekanntmachung des Hinweises auf die Patenterteilung: **10.09.2014**
(21) Anmeldenummer: 08011015.8
(22) Anmeldetag: 18.06.2008
(51) Int. Cl.: A61B 5/151

(54) **Bandkassette für ein medizinisches Handgerät**
Tape cassette for a portable medical device
Cassette à bande pour un appareil médical portable

(43) Veröffentlichungstag der Anmeldung: 23.12.2009
(73) Patentinhaber: Roche Diagnostics GmbH, 68305 Mannheim (DE); F.Hoffmann-La Roche AG, 4070 Basel (CH)
(72) Erfinder: Harttig, Herbert, 67434 Neustadt (DE); Braun, Jürgen, 75365 Calw (DE)
(74) Vertreter: Twelmeier Mommer & Partner

(56) Entgegenhaltungen:
- EP-A- 1 424 040
- WO-A-2004/056269
- WO-A-2005/047861
- WO-A-2005/104948
- WO-A-2008/083844
- US-A- 3 244 287

## Beschreibung

Die Erfindung geht aus von einer Bandkassette mit den im Oberbegriff des Anspruchs 1 angegebenen Merkmalen, wie sie aus der WO 2004/056269 A1 bekannt ist. Die EP 1 424 040 A1 offenbart eine Bandkassette mit koaxial angeordneten Bandrollen, wodurch sich beim Übergang von der einen zur anderen Rolle eine leichte Verdrillung der Trägerbands ergibt.

Derartige Bandkassetten enthalten ein Trägerband mit Funktionselementen, nämlich Testfeldern zur Untersuchung von Körperflüssigkeitsproben und/oder Lanzetten. Zum Gebrauch werden solche Bandkassetten in ein Handgerät eingesetzt, mit dem das Vorhandensein oder die Konzentration eines Analyten, insbesondere die Glukosekonzentration, bestimmt wird und/oder ein Lanzettenstich für eine Probengewinnung durchgeführt wird.

Unbenutzte Funktionselemente von Trägerbändern sind empfindlich, weshalb ein Vorratsabschnitt des Trägerbandes vor schädlichen Umwelteinflüssen geschützt in einer Vorratskammer der Bandkassette angeordnet ist. In dem das Trägerband mit benutzen Funktionselementen auf eine Wickeleinrichtung aufgewickelt wird, wird das Trägerband mit unbenutzten Funktionselementen durch eine Bandaustrittsöffnung der Vorratskammer hindurch gezogen, so dass unbenutzte Funktionselemente nacheinander in eine Gebrauchsposition gelangen.

Bandkassetten mit derartigen Trägerbändem werden insbesondere von Diabetikern benötigt, die mehrmals täglich eine Messung der Glucosekonzentration einer durch einen Lanzettenstich gewonnenen Körperflüssigkeitsprobe, in der Regel Blut oder interstitielle Flüssigkeit, durchführen müssen.

Bekannt sind auch Bandkassetten mit einem Trägerband, das als Funktionselemente sowohl Testfelder als auch Lanzetten trägt. Derartige Bandkassetten sind für integrierte Handgeräte bestimmt, mit denen sowohl eine Messung einer Körperflüssigkeitsprobe als auch ein Lanzettenstich zur Probengewinnung vorgenommen werden können. Einfachere Blutzuckermesssysteme umfassen jedoch häufig zwei separate Handgeräte, nämlich ein Stechgerät und ein Messgerät. Für derartige Stechgeräte sind Bandkassetten bekannt, deren Trägerbänder als Funktionselemente Lanzetten tragen.

Für einen hohen Benutzerkomfort ist bei medizinischen Handgeräten, wie sie insbesondere von Diabetikern verwendet werden, ein möglichst leichter Bandtransport wichtig. Bein einfacheren Handgeräten wird der Bandtransport manuell von dem Benutzer bewirkt, komplexere Handgeräte haben einen Elektromotor, um die Funktionselemente des Trägerbandes nacheinander in eine Gebrauchsposition zu bringen.

Aufgabe der Erfindung ist es deshalb, einen Weg aufzuzeigen, wie bei einem medizinischen Handgerät der Transport eines Trägerbandes erleichtert werden kann.

Diese Aufgabe wird durch eine Bandkassette mit den im Anspruch 1 angegebenen Merkmalen gelöst. Vorteilhafte Weiterbildungen der Erfindung sind Gegenstand der Unteransprüche.

Bei einer im folgenden beschriebenen Bandkassette ist das Trägerband in der Bandaustrittsöffnung quer zu einem in der Vorratskammer angeordneten Vorratsabschnitt orientiert. Dies bedeutet, dass die Bandebene in der Bandaustrittsöffnung gegenüber dem in der Vorratskammer angeordneten Vorratsabschnitt verkippt ist. Überraschender Weise lässt sich durch diese einfach erscheinende Maßnahme eine erhebliche Reibungsreduktion bewirken.

Bevorzugt ist der Vorratsabschnitt auf eine Rolle aufgewickelt, so dass der Vorratsabschnitt parallel zu einer geometrischen Drehachse der Rolle orientiert ist, während ein in der Bandaustrittsöffnung angeordneter Bandabschnitt quer dazu, bevorzugt senkrecht zur geometrischen Drehachse der Rolle, orientiert ist. Der Vorratsabschnitt kann jedoch auch zu einem Stapel gefaltet sein, wobei die Bandebene des in der Bandaustrittsöffnung angeordneten Bandabschnitts quer zur Bandebene des gefalteten Vorratsabschnitts, bevorzugt senkrecht dazu, orientiert sein kann.

Eine Änderung der Orientierung des Trägerbandes wird erfindungsgemäß durch ein Verdrillen des Trägerbandes in der Vorratskammer erreichet, wobei das Trägerband an der Bandaustrittsöffnung aus der Vorratskammer austritt, und die Bandaustrittsöffnung eine an dem Trägerband anliegende Dichtung aufweist. Bevorzugt ist das Trägerband in der Vorratskammer um eine Vierteldrehung verdrillt.

Durch die geänderte Orientierung des Trägerbandes in der Bandaustrittsöffnung lässt sich zudem erreichen, dass sich ein Abschnitt des Trägerbandes mit einem in der Gebrauchsposition positionierten Funktionselement quer zur geometrischen Drehachse der Wickeleinrichtung stellt. In diesem quer stehenden Abschnitt können Lanzetten zum Ausführen eines Stichs in einfacher Weise quer zur geometrischen Drehachse der Wickeleinrichtung bewegt werden. Insbesondere Handgeräte mit einer Stechfunktion können deshalb mit einer in der Bandkassette liegenden Wickeleinrichtung sehr flach konzipiert werden und ergonomisch vorteilhaft in ein an eine Schmalseite des Geräts angelegtes Körperteil, in der Regel einen Finger, stechen.

Bevorzugt trägt das Trägerband zwischen den Testfeldern Lanzetten, so dass die Bandkassette für integrierte Handgeräte geeignet ist, mit denen sowohl eine Messung einer Körperflüssigkeitsprobe als auch ein Lanzettenstich zur Probengewinnung vorgenommen werden kann. Trägt das Trägerband keine Lanzetten, so können die Testfelder in einem Abstand von einander angeordnet werden, aneinander angrenzen oder sogar ineinander übergehen, beispielsweise indem die Testfelder als ein durchgehender Streifen vorgesehen sind.

Weitere Einzelheiten und Vorteile der Erfindung werden an einem Ausführungsbeispiel der Erfindung unter Bezugnahme auf die beigefügte Zeichnung erläutert. Es zeigt:

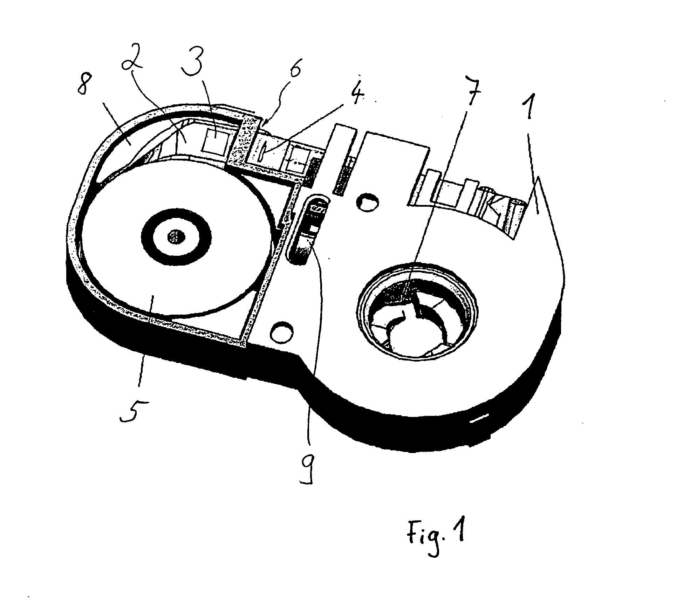

- Figur 1:: ein Ausführungsbeispiel einer erfindungsgemäßen Bandkassette.

Figur 1 zeigt ein Ausführungsbeispiel einer Bandkassette mit teilweise geöffnetem Kassettengehäuse 1. Die Bandkassette enthält ein Trägerband 2, das Testfelder 3 zur Untersuchung einer Probe einer menschlichen oder tierischen Körperflüssigkeit trägt. Bei dem dargestellten Ausführungsbeispiel sind zwischen den Testfeldern 3 Lanzetten 4 angeordnet.

Ein Vorratsabschnitt des Trägerbandes 2 mit unbenutzten Testfeldern 3 ist in einer Vorratskammer angeordnet, in der die Testfelder 2 vor schädlichen Umwelteinflüssen geschützt sind. In Figur 1 ist ein die Vorratskammer verschließender Teil des Gehäuses 1 der Bandkassette entfernt, so dass in der Vorratskammer eine frei drehbare Rolle 5, auf die der Vorratsabschnitt des Trägerbandes 2 aufgewickelt ist, zu sehen ist.

Ein Schutz der unbenutzten Testfelder 2 vor schädlichen Umwelteinflüssen ist wichtig, da Testfelder in der Regel empfindliche Nachweisreagenzien enthalten, beispielsweise für eine photometrische oder elektrochemische Bestimmung einer Analytkonzentration.

Die Vorratskammer hat eine Bandaustrittsöffnung 6, durch die das Trägerband 2 hindurch geführt ist. Benutzte Bandabschnitte werden auf eine Wickeleinrichtung 7 aufgewickelt. Beim Aufwickeln auf die Wickeleinrichtung 7 wird Trägerband 2 aus der Vorratskammer herausgezogen, so dass unbenutzte Testfelder 3 nacheinander für eine Probenaufnahme in eine Gebrauchsposition gebracht werden können. Die Gebrauchsposition liegt auf einer Linie, die senkrecht zu einer Verbindungslinie der geometrischen Drehachse der Rolle 5 und der geometrischen Drehachse der Wickeleinrichtung 7 liegt.

Eine Besonderheit des dargestellten Ausführungsbeispiels ist, dass das Trägerband 2 in der Bandaustrittsöffnung 6 quer zu dem in der Vorratskammer angeordneten Vorratsabschnitt orientiert ist. In der Vorratskammer ist das Trägerband 2 nämlich um eine Vierteldrehung verdrillt, so dass die Ebene des Trägerbandes vor der Bandaustrittsöffnung 6 um 90° gegenüber dem auf die Wickelrolle 5 aufgewickelten Vorratsabschnitt verkippt ist.

Dieses Kippen der Bandebene wird mit einer Bandführung 8 bewirkt, die eine Umlenkschräge aufweist. Die Bandführung 8 kann als ein Trockenmittel enthaltender Kunststoffblock ausgeführt sein. Zur Reibungsreduktion kann die Umlenkschräge eine Oberfläche aus einem Fluorpolymer, beispielsweise aus PTFE oder PVDF, aufweisen.

Die Bandaustrittsöffnung 6 weist eine an dem Trägerband 2 anliegende Dichtung, beispielsweise eine Dichtlippe, auf, um einem Eindringen von Staub oder anderen schädlichen Umwelteinflüssen in die Vorratskammer entgegenzuwirken.

Auf dem Weg von der Bandaustrittsöffnung 6 zu der Wickeleinrichtung 7 wird das Trägerband 2 um eine weitere Vierteldrehung verdrillt. Diese weitere Vierteldrehung kann entgegengesetzt zu der Vierteldrehung in der Vorratskammer erfolgen, also diese wieder rückgängig machen. Bevorzugt erfolgt die weitere Vierteldrehung jedoch in derselben Drehrichtung wie die Vierteldrehung in der Vorratskammer, so dass das Trägerband 2 insgesamt um eine halbe Drehung verdrillt ist.

Bei dem dargestellten Ausführungsbeispiel ist ein in der Gebrauchsposition positioniertes Testfeld 3 mit dem dazugehörenden Trägerbandabschnitt quer zur geometrischen Drehachse der Wickeleinrichtung 7 orientiert.

Die zwischen den Testfeldern 3 auf dem Trägerband 2 angeordneten Lanzetten 4 sind quer zu dessen Längsrichtung orientiert. Auf diese Weise kann ein Antrieb eines Handgeräts, in das die beschriebene Bandkassette eingesetzt wird, eine Probenaufnahme- oder Stichbewegung quer zur Drehachse der Wickeleinrichtung 7 ausführen. Die Bandkassette hat eine Kopplungseinrichtung 9, an die ein Antrieb eines Handgeräts ankoppeln kann, um das Trägerband 2 für eine Probenaufnahme oder einen Lanzettenstich zu bewegen. Bei einer Probenaufnahme oder einer Stechbewegung wird ein in der Gebrauchsposition positioniertes Funktionselemente, also ein Testfeld 3 oder eine Lanzette 4, mit einem es tragenden Bandabschnitt in eine Richtung bewegt, die in der Bandebene des Bandabschnitts liegt und quer seiner Längsrichtung liegt.

Vorteilhaft kann für einen Lanzettenstich oder eine Probenaufnahme im wesentlichen die gleiche Bewegung durchgeführt werden. Bei einer solchen Probenaufnahme- oder Stechbewegung biegt sich die in Bewegungsrichtung vordere Bandkante um, so dass sich eine Lanzettespitze von dem Trägerband 2 abhebt und in ein Körperteil eindringen kann, das an das Handgerät angelegt ist. Ein solches Umbiegen der vorderen Bandkante ist auch für eine Probenaufnahme mit einem in der Gebrauchsposition positionierten Testfeld 3 vorteilhaft, da sich durch das Umbiegen die an dem Körperteil und damit an einer dort austretenden Probe anliegende Fläche vergrößert.

### Bezugszahlen

- 1: Kassettengehäuse
- 2: Trägerband
- 3: Testfelder
- 4: Lanzetten
- 5: Rolle
- 6: Bandaustrittsöffnung
- 7: Wickeleinrichtung
- 8: Bandführung
- 9: Kopplungseinrichtung

## Patentansprüche

1. Bandkassette für ein medizinisches Handgerät, mit
einem Trägerband (2), das als Funktionselemente Testfelder (3) zur Untersuchung einer Probe einer menschlichen oder tierischen Körperflüssigkeit und/oder Lanzetten (4) trägt ,
einer Vorratskammer, in der ein Vorratsabschnitt des Trägerbandes (2) mit unbenutzten Funktionselementen (3, 4) angeordnet ist,
einer Wickeleinrichtung (7), um das Trägerband (2) aufzuwickeln und dabei durch eine Bandaustrittsöffnung (6) der Vorratskammer hindurch zu ziehen, damit die Funktionselemente (3, 4) nacheinander in eine Gebrauchsposition gebracht werden können, wobei, die Bandaustrittsöffnung (6) eine an dem Trägerband (2) anliegende Dichtung aufweist,
**dadurch gekennzeichnet, dass**
das Trägerband (2) in der Vorratskammer verdrillt ist.

2. Bandkassette nach Anspruch 1, **dadurch gekennzeichnet, dass** der Vorratsabschnitt des Trägerbandes (2) auf eine Rolle (5) aufgewickelt ist.

3. Bandkassette nach einem der vorstehenden Ansprüche, **dadurch gekennzeichnet, dass** das Trägerband (2) in der Vorratskammer um eine Vierteldrehung verdrillt ist.

4. Bandkassette nach einem der vorstehenden Ansprüche, **dadurch gekennzeichnet, dass** in der Vorratskammer eine Bandführung (8) mit einer Umlenkschräge angeordnet ist.

5. Bandkassette nach einem der vorstehenden Ansprüche, **dadurch gekennzeichnet, dass** in der Vorratskammer ein Trockenmittel angeordnet ist.

6. Bandkassette nach Anspruch 4 und 5, **dadurch gekennzeichnet, dass** das Trockenmittel in einem Kunststoffblock enthalten ist, der die Bandführung (8) ausbildet.

7. Bandkassette nach einem der Ansprüche 4 bis 6, **dadurch gekennzeichnet, dass** die Umlenkschräge eine Oberfläche aus einem Fluorpolymer aufweist.

8. Bandkassette nach einem der vorstehenden Ansprüche, **dadurch gekennzeichnet, dass** das Trägerband (2) zwischen der Gebrauchsposition und der Wickeleinrichtung (7) um mindestens eine Vierteldrehung verdrillt ist.

9. Bandkassette nach einem der vorstehenden Ansprüche, **dadurch gekennzeichnet, dass** das Trägerband (2) zwischen den Testfeldern (3) Lanzetten (4) trägt.

10. Bandkassette nach Anspruch 9, **dadurch gekennzeichnet, dass** die Wickeleinrichtung (7) um eine geometrische Achse drehbar ist, die quer zu einer in der Gebrauchsposition angeordneten Lanzette (4) verläuft.

## Claims

1. Tape cassette for a medical handheld device, comprising
a carrier tape (2), which carries test fields (3) for assaying a sample of a human and/or animal bodily fluid and/or lancets (4) as functional elements,
a supply chamber, in which a supply section of the carrier tape (2) comprising unused functional elements (3, 4) is positioned,
a winding unit (7), in order to wind up the carrier tape (2) and draw it through a tape exit opening (6) of the supply chamber, so that the functional elements (3, 4) may be brought sequentially into a usage position,
wherein the tape exit opening (6) has a seal touching the carrier tape (2), **characterized in that** the carrier tape (2) is twisted inside the supply chamber.

2. Tape cassette according to claim 1, **characterized in that** the supply section of the carrier tape (2) is wound on a roller (5).

3. Tape cassette according to any one of the preceding claims, **characterized in that** the carrier tape (2) is twisted by a quarter-turn in the supply chamber.

4. Tape cassette according to any one of the preceding claims, **characterized in that** a tape guide (8) having a deflection bevel is positioned in the supply chamber.

5. Tape cassette according to any one of the preceding claims, **characterized in that** a desiccant is positioned in the supply chamber.

6. Tape cassette according to claims 4 and 5, **characterized in that** the desiccant is contained in a plastic block, which forms the tape guide (8).

7. Tape cassette according to one of claims 4 to 6, **characterized in that** the deflection bevel has a surface made of a fluoropolymer.

8. Tape cassette according to any one of the preceding claims, **characterized in that** the carrier tape (2) is twisted by at least a quarter-turn between the usage position and the winding unit (7).

9. Tape cassette according to any one of the preceding claims, **characterized in that** the carrier tape (2) carries lancets (4) between the test fields (3).

10. Tape cassette according to claim 9, **characterized in that** the winding unit (7) is rotatable around a geometric axis, which runs transversely to a lancet (4) positioned in the usage position.

## Revendications

1. Cassette à bande pour un appareil médical portatif, comprenant
une bande support (2) qui porte, en tant qu'éléments fonctionnels, des zones de test (3) destinées à l'examen d'un échantillon d'un fluide corporel humain ou animal et/ou des lancettes (4),
une chambre de réserve, dans laquelle est agencée une section de réserve de la bande support (2) comportant des éléments fonctionnels (3, 4) inutilisés,
un dispositif d'enroulement (7), pour enrouler la bande support (2) et la tirer en même temps au travers d'une ouverture de sortie de bande (6) de la chambre de réserve, afin de pouvoir amener les éléments fonctionnels (3, 4) les uns après les autres dans une position d'utilisation, l'ouverture de sortie de bande (6) présentant un joint en contact avec la bande support (2), **caractérisé en ce que** la bande support (2) est torsadée dans la chambre de réserve.

2. Cassette à bande selon la revendication 1, **caractérisée en ce que** la section de réserve de la bande support (2) est enroulée sur un rouleau (5).

3. Cassette à bande selon l'une quelconque des revendications précédentes, **caractérisée en ce que** la bande support (2) est torsadée d'un quart de tour dans la chambre de réserve.

4. Cassette à bande selon l'une quelconque des revendications précédentes, **caractérisée en ce qu'**un guide-bande (8) comprenant un élément déflecteur oblique est agencé dans la chambre de réserve.

5. Cassette à bande selon l'une quelconque des revendications précédentes, **caractérisée en ce qu'**un agent déshydratant est agencé dans la chambre de réserve.

6. Cassette à bande selon les revendications 4 et 5, **caractérisée en ce que** l'agent déshydratant est contenu dans un bloc de matière plastique qui constitue le guide-bande (8).

7. Cassette à bande selon l'une quelconque des revendications 4 à 6, **caractérisée en ce que** l'élément déflecteur oblique présente une surface réalisée à partir d'un polymère fluoré.

8. Cassette à bande selon l'une quelconque des revendications précédentes, **caractérisée en ce que** la bande support (2) est torsadée d'au moins un quart de tour entre la position d'utilisation et le dispositif d'enroulement (7).

9. Cassette à bande selon l'une quelconque des revendications précédentes, **caractérisée en ce que** la bande support (2) porte des lancettes (4) entre les zones de test (3).

10. Cassette à bande selon la revendication 9, **caractérisée en ce que** le dispositif d'enroulement (7) peut être pivoté autour d'un axe géométrique positionné de manière transversale par rapport à une lancette (4) agencée dans la position d'utilisation.
